(19) Europäisches Patentamt
European Patent Office
Office européen des brevets

(11) Veröffentlichungsnummer: **0 237 762**
**B1**

(12) **EUROPÄISCHE PATENTSCHRIFT**

(45) Veröffentlichungstag der Patentschrift: **07.06.89**

(51) Int. Cl.⁴: **C25B 3/02**, C07D 231/12

(21) Anmeldenummer: **87101734.9**

(22) Anmeldetag: **09.02.87**

(54) **Verfahren zur Herstellung von Pyrazolen.**

(43) Veröffentlichungstag der Anmeldung:
**23.09.87 Patentblatt 87/39**

(45) Bekanntmachung des Hinweises auf die Patenterteilung:
**07.06.89 Patentblatt 89/23**

(84) Benannte Vertragsstaaten:
**CH DE FR GB IT LI NL**

(56) Entgegenhaltungen:
**US-A- 4 434 292**

**ZEITSCHRIFT FÜR CHEMIE, 14. Jahrgang, Heft 6, 1974, Leipzig. F. PRAGST "Anodische Oxydation von N-Aryl-Delta2-pyrazolinen zu Pyrazolen" Seiten 236, 237**
**CHEMICAL ABSTRACTS; Band 81, Nr. 14, 7. Oktober 1974, Columbus, Ohio, USA. F. PRAGST et al. "Electrochemical behavior of N-aryl-2-pyrazolines" Seite 511, Spalte 2, Zusammenfassung-Nr. 85 114k**
**CHEMICAL ABSTRACTS; Band 99, Nr. 25, 19. Dezember 1983, Columbus, Ohio, USA. F. LECOUTRE et al. "Electrochemical oxidation of 3-aminopyrazolines" Seite 594, Spalte 1, Zusammenfassung-Nr. 211 948x**

(73) Patentinhaber: **BASF Aktiengesellschaft, Carl-Bosch-Strasse 38, D-6700 Ludwigshafen(DE)**

(72) Erfinder: **Steiniger, Michael, Dr., Wolfskeule 1, D-6730 Neustadt(DE)**
Erfinder: **Stroefer, Eckhard, Dr., Werderstrasse 20, D-6800 Mannheim 1(DE)**

**EP 0 237 762 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von Pyrazolen durch elektrochemische Oxidation von 2-Pyrazolinen.

Pyrazole lassen sich bekanntlich durch Oxidation von 2-Pyrazolinen herstellen, wobei man als Oxidationsmittel z.B. Brom (Adv. Heterocycl. Chem. 6, 385 (1966)), Chlor und Hypochlorite (EP-B-48 373) oder Kaliumpermanganat, Salpetersäure, Luft, Bleitetraacetat oder Chromtrioxid (The Chemistry of Heterocyclic Compounds, Band 22, S. 41-49 (1967)) sowie Peroxide (EP-A-162 247) verwendet. Die Dehydrierung von Pyrazolinen zu den entsprechenden Pyrazolen gelingt nach den Angaben der EP-B-45 394 und der DE-A-32 09 148 mit Schwefel bzw. Selen oder an Katalysatoren der Platinmetalle in der Gasphase.

Nachteilig bei diesen bekannten Verfahren ist der Einsatz teurer und/oder toxischer Oxidationsmittel, die Entsorgung der Produkte, die aus den Oxidationsmitteln entstehen, sowie die aus sicherheitstechnischen Gründen aufwendige Reaktionsführung. Es war deshalb die Aufgabe gestellt, ein Verfahren zur Herstellung von Pyrazolen zu finden, das technisch einfacher und ökonomischer ausgeführt werden kann und sich durch besondere Umweltfreundlichkeit auszeichnet.

Es wurde nun gefunden, daß man Pyrazole der allgemeinen Formel I

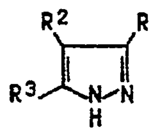

I,

in der die Reste $R^1$ bis $R^3$ Wasserstoffatome, Alkylreste, Cycloalkylreste oder Arylreste bedeuten, besonders vorteilhaft herstellen kann, wenn man 2-Pyrazoline der allgemeinen Formel II

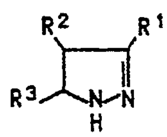

II,

in der $R^1$ bis $R^3$ die oben genannte Bedeutung haben, in Gegenwart eines ionogenen Halogenids elektrochemisch oxidiert.

Die Reaktion des erfindungsgemäßen Verfahrens läßt sich durch die folgenden Formeln beschreiben:

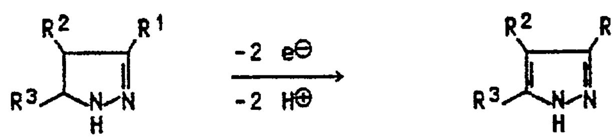


(II) (I)

Das vorteilhafte Ergebnis des erfindungsgemäßen Verfahrens ist überraschend, da sich bislang nur solche 2-Pyrazoline anodisch zu Pyrazolen oxidieren ließen, welche zur Stabilisierung des intermediär entstehenden Radikalkations eine N-Arylgruppe tragen. Auch dabei gelang die elektrochemische Oxidation von z.B. 1-Phenyl-2-pyrazolin zum entsprechenden Pyrazol nur in Gegenwart einer Base, wie Pyridin. Ohne Basenzusatz wurde das Dimere Bis-[1-phenyl-2-pyrazolinyl-3-] gebildet (Z. Chem. 14, 236 (1974) oder Adv. Heterocyclic Chem., 36, 237 (1984)).

Die als Ausgangsstoffe verwendeten 2-Pyrazoline der Formel II enthalten als Reste $R^1$ bis $R^3$ Wasserstoffatome, Alkyl-, Cycloalkyl- und Arylreste. Die Alkylreste enthalten z.B. 1 bis 12, vorzugsweise 1 bis 8, insbesondere 1 bis 4 Kohlenstoffatome, wie Methyl, Ethyl-, n- und iso-Propyl, n-Butyl- oder tert.-Butylreste. Als Cycloalkylreste kommen z.B. solche mit 3 bis 8, insbesondere mit 5 und 6 Kohlenstoffatomen in Betracht. Arylreste sind z.B. Phenylgruppen. Die Arylreste können noch unter den Reaktionsbedingungen inerte Substituenten tragen, wie Alkyl- oder Alkoxigruppen mit bis 4 C-Atomen, Halogen oder Nitrilgruppen.

Beispielsweise seien die folgenden Verbindungen der Formel II genannt: 2-Pyrazolin, 3-Methyl-2-pyrazolin, 4-Phenyl-2-pyrazolin, 4-Methyl-2-pyrazolin, 4,5-Dimethyl-2-pyrazolin.

Die Ausgangsverbindungen der Formel II lassen sich z.B. auf bekannte Weise durch Umsetzung von Hydrazin mit α,β-ungesättigten Aldehyden wie Acrolein oder entsprechend substituierten Acroleinen herstellen.

Als ionogene Halogenide kommen Salze der Jodwasserstoff-, Bromwasserstoff- und Chlorwasserstoffsäure oder die genannten Säuren selbst in Betracht. Besonders bevorzugt sind Salze der Bromwasserstoffsäure, wie Alkali- oder Erdalkalibromide sowie quaternäre Ammonium-, insbesondere Tetraalkylammoniumbromide. Das Kation spielt keine erfindungswesentliche Rolle, es können daher auch andere ionogene Metallhalogenide verwendet werden, zweckmäßigerweise wird man jedoch billige Halogenide wählen. Beispielsweise seien Natrium-, Kalium-, Calcium- und Ammoniumbromid sowie Di-, Tri- und Tetramethyl- oder Tetraethylammoniumbromid genannt. Für die Elektrooxidation verwendet man zweckmäßigerweise eine Lösung der Pyrazoline und des ionogenen Halogenids in einem geeigneten Lösungsmittel. Als Lösungsmittel eignen sich solche, die sich unter den Elektrolysebedingungen als ausreichend stabil erweisen. Das sind z.B. Ether, wie Tetrahydrofuran, Chlorkohlenwasserstoffe, wie Methylenchlorid, Ketone, wie Aceton und Nitrile, wie Acetonitril. Besonders geeignet sind Alkohole, wie Methanol und Ethanol.

Die Zusammensetzung des Elektrolyten kann in weiten Grenzen gewählt werden. So besteht der Elektrolyt beispielsweise aus einer alkoholischen Lösung des 2-Pyrazolins der allgemeinen Formel II mit einem Gehalt an 2-Pyrazolin von 1 bis 30, vorzugsweise 3 bis 15 Gew.% und einem Gehalt an dem ionogenen Halogenid von 0,1 bis 20, vorzugsweise 1 bis 10 Gew.%.

Das erfindungsgemäße Verfahren kann in den üblichen technischen Elektrolysezellen durchgeführt werden. Man verwendet vorzugsweise ungeteilte Durchflußzellen, die zweckmäßigerweise zur Minimierung der Zellspannung geringe Elektrodenabstände von beispielsweise 0,25 bis 2 mm ermöglichen. Als Anoden werden Edelmetalle, mit Edelmetalloxiden dotiertes Titan, Metalloxide, wie $MnO_2$ und $PbO_2$, bevorzugt jedoch Graphit eingesetzt. Die Kathodenmaterialien sind z.B. Blei, Eisen, Stahl, Nickel oder Edelmetalle wie Platin. Bevorzugtes Kathodenmaterial ist ebenfalls Graphit.

Die Elektrolyse wird z.B. mit 1 bis 4 F/Mol, vorzugsweise 1,5 bis 3 F/Mol des 2-Pyrazolins durchgeführt. Die Stromdichte ist kein begrenzender Faktor, sie beträgt z.B. 0,1 bis 25 A/dm$^2$, vorzugsweise 3 bis 15 A/dm$^2$. Man führt das erfindungsgemäße Verfahren z.B. in einem Temperaturbereich zwischen 10°C und einer Temperatur, die 5°C unter dem Siedepunkt des eingesetzten Lösungsmittels liegt, durch. Bei Verwendung von Methanol oder Ethanol wird bei druckloser Fahrweise bei Temperaturen von 10 bis 40°C elektrolysiert. Die Elektrolyse kann sowohl diskontinuierlich als auch kontinuierlich durchgeführt werden.

Die Aufarbeitung der Elektrolyseausträge kann man nach an sich bekannten Methoden vornehmen. Zweckmäßigerweise wird der Elektrolyseaustrag destillativ aufgearbeitet. Überschüssiges Lösungsmittel wird zunächst abdestilliert. Die Halogene werden in bekannter Weise, z.B. durch Filtration oder Extraktion abgetrennt und das Produkt reindestilliert bzw. umkristallisiert. Lösungsmittel, evtl. unumgesetztes 2-Pyrazolin sowie Halogenide können vorteilhaft zur Elektrolyse rückgeführt werden.

Die nach dem erfindungsgemäßen Verfahren zugänglichen Pyrazole der allgemeinen Formel I sind vielfältig einsetzbare Zwischenprodukte für die Synthese von Farbstoffen, Pharmazeutika und Pflanzenschutzmitteln.

Beispiele

Die Elektrooxidationen wurden in einer ungeteilten Elektrolysezelle mit 6 Graphitelektroden (5 Spalte, Elektrodenabstand 0,5 bis 1 mm) durchgeführt. Während der Elektrolyse wurde der Elektrolyt, dessen Zusammensetzung den Beispielen 1 bis 7 zu entnehmen ist, mit 200 l/h über einen Wärmetauscher durch die Zelle gepumpt.

Beispiel 1

Eine Lösung von 117g 2-Pyrazolin und 64 g NaBr in 3000g Methanol wurde bei einer Stromdichte von 4,8 A/dm$^2$ und einer Temperatur von 10°C mit 2 F/Mol 2-Pyrazolin elektrolysiert. Die gaschromatographische Analyse zeigte, daß im Elektrolyseaustrag 62 g Pyrazol und 18,6 g unumgesetztes 2-Pyrazolin enthalten waren. Hieraus errechnet sich ein Umsatz von 83%, eine Ausbeute von 55 % und eine Selektivität von 65%. Vom Elektrolyseaustrag wurde Methanol bei Normaldruck bis zu einer Sumpftemperatur von 100 bis 110°C abdestilliert. Der Rückstand wurde von Bromid befreit und bei 90 bis 105°C und 50 mbar reindestilliert. Es konnten 16 g 2-Pyrazolin und 58,1 g Pyrazol, entsprechend einer Ausbeute von 50 % und einer Selektivität von 58 %, isoliert werden.

Beispiel 2

Eine Lösung von 295 g 2-Pyrazolin und 53 g NaBr in 2600 g Methanol wurde bei einer Stromdichte von 6,8 A/dm$^2$ und einer Temperatur von 20°C mit 2 F/Mol 2-Pyrazolin elektrolysiert. Aus der gaschromatographischen Analyse errechnete sich ein Umsatz von 84 %, eine Ausbeute von 50 % und eine Selektivität von 61 %.

Beispiel 3

117 g 2-Pyrazolin und 64 g KBr wurden in 3000 g Methanol gelöst, bei einer Stromdichte von 9 A/dm$^2$ und einer Temperatur von 15°C mit 2 F/Mol 2-Pyrazolin elektrolysiert. Die gaschromatographische Analyse des Elektrolyseaustrags ergab 9,5 g an unumgesetztem 2-Pyrazolin und 66 g Pyrazol. Hieraus errechnet sich ein Umsatz von 92 %, eine Ausbeute von 58 % und eine Selektivität von 63 %.

Beispiel 4

Eine Lösung von 117 g 2-Pyrazolin und 64 g Tetraethylammoniumbromid in 3000 g Methanol wurde bei einer Stromdichte von 9 $A/dm^2$ und einer Temperatur von 40°C mit 2 F/Mol 2-Pyrazolin elektrolysiert. Die gaschromatographische Analyse des Austrages ergab 30,8 g 2-Pyrazolin und 68 g Pyrazol. Hieraus errechnet sich eine Ausbeute von 60 % an Pyrazol und bei einem Umsatz von 74 % eine Selektivität von 81 %. Durch fraktionierte Destillation wurden bei 99°C und 50 mbar 66 g Pyrazol (58 % Ausbeute; 78 % Selektivität) isoliert.

Beispiel 5

In 3000 g Ethanol wurden 234 g 2-Pyrazolin und 64 g Tetraethylammoniumbromid gelöst und bei einer Stromdichte von 6,8 $A/dm^2$ und einer Temperatur von 30°C mit 2 F/Mol 2-Pyrazolin elektrolysiert. Die zu Beispiel 1 analoge Aufarbeitung ergab 18,2 g 2-Pyrazolin und 129 g Pyrazol. Hieraus errechnet sich ein Umsatz von 92 %, eine Ausbeute von 57 % und eine Selektivität von 62 %.

Beispiel 6

Eine Lösung von 100 g 3-Methyl-2-pyrazolin und 64 g NaBr in 3000 g Methanol wurde bei einer Stromdichte von 6,8 $A/dm^2$ und einer Temperatur von 20°C mit 2 F/Mol 3-Methyl-2-pyrazolin elektrolysiert. Übliche Aufarbeitung des Elektrolyseaustrags und fraktionierte Destillation ergab 24,5 g 3-Methyl-2-pyrazolin und 29,5 g 3-Methylpyrazol (Sdp. 127 bis 131°C/98 mbar). Hieraus errechnet sich ein Umsatz von 75 %, eine Ausbeute von 30 % und eine Selektivität von 40 %.

Beispiel 7

Eine Lösung von 146 g 4-Phenyl-2-pyrazolin und 64 g KBr in 3000 g Methanol wurde bei einer Stromdichte von 9 $A/dm^2$ und einer Temperatur von 20°C mit 2 F/Mol 4-Phenyl-2-pyrazolin elektrolysiert. Durch gaschromatographische Analyse wurde festgestellt, daß im Elektrolyseaustrag 28,5 g 4-Phenyl-2-pyrazolin und 72 g 4-Phenylpyrazol enthalten waren. Die Ausbeute an 4-Phenylpyrazol beträgt demnach 50 %, die Selektivität bei einem Umsatz von 80 % beträgt 63 %.

**Patentansprüche**

1. Verfahren zur Herstellung von Pyrazolen der allgemeinen Formel

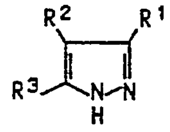

I,

in der $R^1$ bis $R^3$ Wasserstoffome, Alkylreste, Cycloalkylreste oder Arylreste bedeuten, dadurch gekennzeichnet, daß man 2-Pyrazoline der allgemeinen Formel

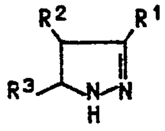

II,

in der $R^1$ bis $R^3$ die oben genannte Bedeutung haben, in Gegenwart eines ionogenen Halogenids elektrochemisch oxidiert.

2. Verfahren nach Anspruch 1, dadurch gekennzeichnet, daß man als ionogenes Halogenid ein Salz der Chlor- oder Bromwasserstoffsäure verwendet.

3. Verfahren nach Anspruch 1, dadurch gekennzeichnet, daß man für die Elektrolyse Graphitanoden verwendet.

4. Verfahren nach Anspruch 1, dadurch gekennzeichnet, daß man für die Elektrooxidation eine Lösung des 2-Pyrazolins und des ionogenen Halogenids in einem unter den Elektrolysebedingungen stabilen Lösungsmittel verwendet.

5. Verfahren nach Anspruch 4, dadurch gekennzeichnet, daß man eine alkoholische Lösung des 2-Pyrazolins verwendet, die 1 bis 30 Gew.% des 2-Pyrazolins und 0,1 bis 20 Gew.% des Halogenids enthält.

6. Verfahren nach den Ansprüchen 4 bis 5, dadurch gekennzeichnet, daß man als Lösungsmittel Ether, Chlorkohlenwasserstoffe, Ketone, Nitrile oder Alkohole verwendet.

7. Verfahren nach den Ansprüchen 4 bis 6, dadurch gekennzeichnet, daß man als Lösungsmittel Methanol oder Ethanol verwendet.

8. Verfahren nach Anspruch 1, dadurch gekennzeichnet, daß man die Elektrolyse mit 1 bis 4 F/Mol des 2-Pyrazolins durchführt.

9. Verfahren nach Anspruch 1, dadurch gekennzeichnet, daß man bei Stromdichten von 0,1 bis 25 A/dm² elektrolysiert.

10. Verfahren nach den Ansprüchen 4 bis 9, dadurch gekennzeichnet, daß man die Elektrolyse in einem Temperaturbereich zwischen 10°C und einer Temperatur, die 5°C unter dem Siedepunkt des eingesetzten Lösungsmittels liegt, durchführt.

## Claims

1. A process for preparing a pyrazole of the general formula

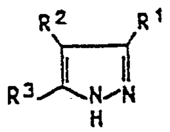

I

where $R^1$, $R^2$ and $R^3$ are each hydrogen, alkyl, cycloalkyl or aryl, which comprises electrochemically oxidizing a 2-pyrazoline of the general formula

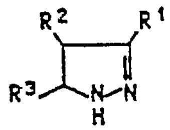

II

where $R^1$, $R^2$ and $R^3$ have the abovementioned meanings, in the presence of an ionic halide.

2. A process as claimed in claim 1, wherein the ionic halide is a salt of hydrochloric or hydrobromic acid.

3. A process as claimed in claim 1, wherein the electrolysis is performed with graphite anodes.

4. A process as claimed in claim 1, wherein the electrooxidation is performed on a solution of the 2-pyrazoline and the ionic halide in a solvent which is stable under the electrolysis conditions.

5. A process as claimed in claim 4, wherein the 2-pyrazoline is used in the form of an alcoholic solution which contains from 1 to 30% by weight of the 2-pyrazoline and from 0.1 to 20% by weight of the halide.

6. A process as claimed in claim 4 or 5, wherein the solvent is an ether, a hydrocarbon, a ketone, a nitrile or an alcohol.

7. A process as claimed in claims 4 to 6, wherein the solvent is methanol or ethanol.

8. A process as claimed in claim 1, wherein the electrolysis is carried out with from 1 to 4 F/mol of 2-pyrazoline.

9. A process as claimed in claim 1, wherein the electrolysis is carried out at current densities of from 0.1 to 25 A/dm².

10. A process as claimed in claims 4 to 9, wherein the electrolysis is carried out within a temperature range of from 10°C below the boiling point of the solvent used.

## Revendications

1. Procédé de préparation de pyrazoles de formule générale

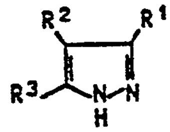

I.

dans laquelle $R^1$ à $R^3$ sont des atomes d'hydrogène ou des groupements alkyle, cycloalkyle ou aryle, caractérisé en ce qu'on oxyde par voie électrochimique une 2-pyrazoline de formule

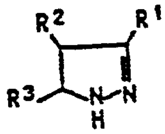

II.

dans laquelle R1à R3 sont tels que définis ci-dessus, en présence d'un halogénure ionogène.

2. Procédé selon la revendication 1, caractérisé en ce qu'on utilise comme halogénure ionogène un sel d'acide chlorhydrique ou bromhydrique.

3. Procédé selon la revendication 1, caractérisé en ce qu'on utilise pour l'électrolyse des anodes en graphite.

4. Procédé selon la revendication 1, caractérisé en ce qu'on utilise pour l'électro-oxydation une solution de la 2-pyrazoline et de l'halogénure ionogène dans un solvant stable dans les conditions de l'électrolyse.

5. Procédé selon la revendication 4, caractérisé en ce qu'on utilise une solution alcoolique de la 2-pyrazoline qui contient de 1 à 30% en poids de la 2-pyrazoline et de 0,1 à 20% en poids de l'halogénure.

6. Procédé selon l'une ou l'autre des revendications 4 et 5, caractérisé en ce qu'on utilise comme solvant un éther, un hydrocarbure chloré, une cétone, un nitrile ou un alcool.

7. Procédé selon l'une quelconque des revendications 4 à 6, caractérisé en ce qu'on utilise comme solvant du méthanol ou de l'éthanol.

8. Procédé selon la revendication 1, caractérisé en ce qu'on effectue l'électrolyse avec 1 à 4 F par mole de 2-pyrazoline.

9. Procédé selon la revendication 1, caractérisé en ce qu'on électrolyse avec des densités de courant de 0,1 à 25 A/dm2.

10. Procédé selon l'une quelconque des revendications 4 à 9, caractérisé en ce qu'on effectue l'électrolyse dans une gamme de température comprise entre 10°C et une température située à 5°C en dessous du point d'ébullition du solvant utilisé.